(19) Europäisches Patentamt
European Patent Office
Office européen des brevets

(11) Publication number: 0 177 696
A1

(12) # EUROPEAN PATENT APPLICATION

(21) Application number: 85109538.0

(22) Date of filing: 29.07.85

(51) Int. Cl.⁴: C 07 H 17/08

(30) Priority: 31.08.84 US 646558

(43) Date of publication of application: 16.04.86
Bulletin 86/16

(84) Designated Contracting States: AT BE CH DE FR GB IT LI LU NL SE

(71) Applicant: ABBOTT LABORATORIES, 14th Street and Sheridan Road North St, North Chicago Illinois 60064 (US)

(72) Inventor: Pariza, Richard J., 420 Russel Avenue, Winthrop Harbor Illinois 60096 (US)
Inventor: Freiberg, Leslie A., 10066 W. Hendee, Waukegan Illinois 60087 (US)

(74) Representative: Modiano, Guido et al, MODIANO, JOSIF, PISANTY & STAUB Modiano & Associati Via Meravigli, 16, I-20123 Milan (IT)

(54) **Macrolide alkylation process.**

(57) O-Alkylated derivatives of macrolide antibiotics which exhibit improved antibiotic activity can be prepared from parent macrolides having a plurality of reactive hydroxyl groups via a two-step process involving, first, protective esterification of the hydroxy-amino sugar moiety, followed by alkylation at one or more of the remaining hydroxyl groups.

EP 0 177 696 A1

0177696

# MACROLIDE ALKYLATION PROCESS

## Technical Field

This invention relates to a new synthetic process for the preparation of O-alkylated macrolide antibiotics. In particular, it relates to the preparation of alkylated erythromycin derivatives.

## Background Art

O-alkylated derivatives of erythromycin and closely related antibiotics such as oleandomycin are known which have been stated to have a desirable combination of enhanced acid stability and improved antibacterial activity versus the present erythromycin compound. For example, U. S. Patent 4,331,803, issued May 25, 1982, to Watanabe et al. and assigned to Taisho Pharmaceutical Co. Ltd., describes 6-O-methyl erythromycin A derivatives which are stated to have improved antibiotic activity in comparison to erythromycin. However, the process described in the '803 patent for the preparation of those compounds involved protection of the 2'-O- and the 3'-dimethylamino- positions. That was done by adding carbobenzoxy groups at those positions, removing one of the methyl groups on the amino function in the process. Following nonselective O-methylation of the compound, the carbobenzoxy protecting groups were removed and the methyl group was reintroduced. That synthetic process is long and difficult, and results in low overall yields. As a result, faster, more efficient processes with high yields and higher purity are still sought for economical production of O-alkylated macrolides.

Disclosure of the Invention

This invention provides a shorter and much less expensive route to the preparation of O-alkylated macrolide antibiotics, such as erythromycin, than is taught in the art. The overall yields are higher, the purity is improved, and the synthetic process can be extended to new alkylated macrolides, as well as those taught in the art.

This invention is based on the discovery that protection of the most reactive hydroxyl group (the 2'-hydroxyl in the cases of erythromycin, oleandomycin, and derivatives thereof) by simple esterification provides adequate protection of the adjacent dimethylamine group, and prevents quaternization of that amine when subsequent O-alkylation is carried out at one or more of the remaining hydroxyl groups. As a result, the deprotection and re-methylation steps are avoided. The ester can be removed later, if desired, by simple hydrolysis.

In general, this invention provides a process for O-alkylation of a macrolide antibiotic having a plurality of reactive hydroxyl groups, at positions other than the most reactive hydroxyl group, comprising esterifying the most reactive hydroxyl group, followed by alkylating one or more of the remaining hydroxyl groups.

In a preferred embodiment, this invention provides a method for producing 6-O-, 11-O- and 6,11-di-O-alkylated erythromycin derivatives comprising esterifying the 2'-hydroxyl group with an acylating agent selected from compounds of the formulas:

(A) $$X\text{-}R\text{-}\overset{\displaystyle O}{\overset{\|}{C}}\text{-}Y$$

where Y is halogen, such as fluoride, chloride or bromide; R is divalent alkyl, hydroxyalkyl, cycloalkyl or heterocyclic alkyl of 1 to 22 carbon atoms, or alkenyl, aryl, heteroaryl, or alkaryl of 3 to 18 carbon atoms and X is hydrogen or a terminal acyl group of the formula:

$$R'-O-\overset{O}{\overset{\|}{C}}-$$

where R' is monovalent alkyl of 1 to 3 carbon atoms; and

(B) $(R^2-C)_2O$

wherein $R^2$ is monovalent alkyl, cycloalkyl, hydroxyalkyl, or heterocyclic alkyl of 1 to 22 carbon atoms, alkenyl, aryl, heteroaryl or alkaryl of 3 to 18 carbon atoms, followed by O-alkylation with an alkyl halide in the presence of a strong base.

The term "alkyl of 1 to 22 carbon atoms" implies esterification with, for example, the $C_1$ to $C_{22}$ fatty acids and diacids, such as acetic propionic valeric, caproic, caprylic, capric, lauric, myristic, palmitic, stearic, arachidic, malonic sebacic, azelaic, suberic, and succinic acids, as well as the methyl, ethyl and propyl monoesters of the latter dicarboxylic acids. The term "cycloalkyl" includes, for example, 4-cyclohexyl, 6-cyclopropyl and 2-norbornyl hexadecyl groups. The term "heterocyclic alkyl" includes, for example, the morpholino, piperidino, piperazino, epoxy and dioxol groups. The term "alkenyl of 3 to 18 carbon atoms" implies esterification with, for example, 2-propenoic, sorbic, palmitoleic, maleic, fumaric, oleic and myristoleic acids. The term "hydroxyalkyl of 1 to 22 carbon atoms" includes esterification with common hydroxycarboxylic acids, such as lactic, lactobionic, ascorbic, gluconic, malic, tartaric and tartronic acids.

In general, the esterification of the macrolide can be accomplished by using any of the variety of techniques and reagents described in the literature, including H. W. Murphy, Antibiot. Annu., 1953-1954, 500 (1954); V. C. Stephens, id., 514 (1954); R. V. Clark and E. L. Varnes, Antibiot. Chemother., 7, 487 (1957); V. C. Stephens and J. W. Corrine, Antibiot. Annu., 1958-1959, 346 (1959), and U.S. 4,069,379 issued January 17, 1978 to Sciavolino, the disclosures of all of which are hereby incorporated herein by reference. These techniques can be applied to esterify the most reactive hydroxyl group with the preferred esterifying groups of this invention, which are saturated and unsaturated fatty acids of from 1 to 18 carbon atoms, and dicarboxylic acids and dicarboxylic acid esters of from 3 to 12 carbon atoms. Most preferred esterifying groups are short chain ($C_1$ to $C_6$) aliphatic carboxylic acids, as well as succinic acid and $C_1$ to $C_3$ monoesters thereof.

Following esterification of the most reactive hydroxyl group, the macrolide esters are alkylated at one or more of the remaining hydroxyl groups by any convenient alkylation reaction. Any of the macrolide O-alkylation techniques disclosed in the literature can be used. One such technique involves the use of an alkyl halide with a strong base such as an alkali metal hydride. Among the alkali metal hydrides useful in this invention are sodium hydride and potassium hydride. A preferred alkali metal hydride is sodium hydride. Other preferred bases include sodium dimsyl, sodium silazide and lithium silazide. Alkyl halides include the alkyl chlorides, bromides and iodides. A preferred halide is the iodide, because of its relatively higher reactivity rate as a leaving group in the alkylation reaction. Alkyl sulfonates, methane sulfonates and fluoromethane

sulfonates can also be used. The alkyl moiety can be a $C_1$ to $C_6$ straight- or branched-chain alkyl group, preferably a $C_1$ to $C_3$ n-alkyl, and is most preferably a methyl group. As stated, no protection of the tertiary amino group is necessary in this reaction step.

In a preferred embodiment, this process involves alkylation of the 6'-O-, or 11'-O- position, or both positions, of erythromycin. Depending upon conditions, 6-O-alkylated, 11-O-alkylated, and 6,11-di-O-alkylated erythromycin derivatives can be prepared.

A number of the resulting alkylated macrolide ester compounds have antimicrobial activity per se and can be used as such. Alternatively, the compounds can be used as intermediates for further reaction to yield derivatives of the 6-O-alkylated, 11-O-alkylated and 6,11-di-O-alkylated macrolide esters. Finally, the ester group can be removed by hydrolysis when subsequent processing conditions do not require further protection of the OH- group or the adjacent amine, to yield 6-O-alkylated 11-O-alkylated, and 6,11-di-O-alkylated macrolide compounds, and derivatives thereof. This reaction process can be illustrated further by reference to the following non-limiting examples:

Example I

Reaction of 2'-Acetylerythromycin A With Sodium Hydride/Methyl Iodide

A solution of 5.00 g (6.45 mmol) of 2'-acetyl-erythromycin A in a mixture of 18 ml day dimethylsulfoxide (DMSO) and 54 ml of dry tetrahydrofuran (THF) was cooled while stirring in a refrigerated methanol bath at -27° C. 3.8 ml (61.0 mmol) of methyl iodide was added to the stirred, partially crystallized mixture, followed immediately by

0.387 g (8.06 mmol) of sodium hydride (50% dispersion in mineral oil). After 3 hours 17 ml (121.5 mmol) of triethylamine (TEA) was added and the mixture was stirred for 1 hour in an ice bath to destroy excess methyl iodide. The reaction mixture was then poured into 300 ml of toluene. The toluene mixture was washed with two 100 ml portions of 4% sodium bicarbonate solution. The toluene layer was dried over anhydrous sodium sulfate, filtered and concentrated to dryness using a rotary evaporator to yield a glass. The resulting glass was dried in vacuo overnight to yield 3.76 g of crude product which was found to be a mixture of polar and nonpolar components by thin layer chromatography. A 3.14 g portion of this mixture was resolved by chromatography on a 200 g column (3.0 x 60 cm) of Silica Gel 60 (70-230 mesh ASTM, E. Merck, Darmstadt, Germany) prepared in acetonitrile and then equilibrated with the eluent $CHCl_3$:25% $CH_3CN$:0.5% concentrated $NH_4OH$. The column was run at a flow rate of 2.6 ml/minute and fractions were cut at 9 minute intervals. On the basis of thin layer chromatographic results, appropriate fractions were combined and the solvent was removed with a rotary evaporator. Fractions 31 - 43 provided 1.14 g of the less polar component which by 'H-nmr proved to be a 3:1 mixture of 6-O- and 6,11-di-O-methylated products. The mixture was crystallized twice from methanol (-25°C) to yield 2'-acetyl-6-O-methylerythromycin A as needles: mp. 259 - 264° C.

Anal calcd for $C_{40}H_{71}NO_{14}$ (790.010): C, 60.82; H, 9.06; N, 1.77.

Found: C, 60.52; H, 9.06; N, 1.77.

Fractions 54 - 90 provided 1.0 g of the more polar component which was crystallized from acetonitrile to give 328 mg of 2'-acetyl-11-O-methylerythromycin A as

a mixture of 9-keto and 6,9-hemiketal monohydrate forms: mp. 168 - 171° C.

Anal calcd for $C_{40}H_{73}NO_{15}$ (808.026): C, 59.46; H, 9.11; N, 1.73.

Found: C, 59.67; H, 8.91; N, 1.47.

Example II

If in the process of Example I a larger quantity of sodium hydride is used (e.g., 2 to 2.5 equivalents), then 2'-acetyl-6,11-di-O-methylerythromycin A can be isolated by crystallization from acetonitrile: m.p. 252-257°.

Example III

Reaction of 2'-ethylsuccinylerythromycin A With Sodium Hydride/Methyl Iodide

A 5.00 g (5.80 mmol) sample of 2'-ethylsuccinylerythromycin A was O-methylated in a manner identical to Example I. The crude product mixture (3.68 g) was chromatographed according to the procedure of Example I.

Upon evaporation of the eluent, fractions 27 - 44 provided 1.09 g of the less polar component which was crystallized from benzene:heptane to give 2'-ethylsuccinyl-6-O-methylerythromycin A as needles: mp. 215 - 220° C.

Anal calcd for $C_{44}H_{77}NO_{16}$ (876.101): C, 60.32; H, 8.86; N, 1.60.

Found: C, 60.63; H, 8.86; N, 1.35.

On evaporation of the solvent, fractions 54 - 90 gave 1.11 g of the more polar component which could not be crystallized. The material was dissolved in 40 ml of acetonitrile and treated with 0.5 g of charcoal. The charcoal was removed by filtration on Whatman No. 42 paper and the solvent was evaporated. The residue was triturated with heptane and the solid 2'-ethylsuccinyl-11-O-methylerythromycin was isolated as

a mixture of 9-keto and 6,9-hemiketal forms.

Anal calcd for $C_{44}H_{77}NO_{16}$ (876.101): C, 60.32; H, 8.86; N, 1.60.

Found: C, 60.10; H, 8.85; N, 1.60.

## Example IV

As in Example II if larger quantities of sodium hydride are used then 2'-ethylsuccinyl-6,11-di-O-methylerythromycin A can be isolated from the non-polar column fractions by crystallization from acetonitrile.

## Example V

### Alkylation of 2'-acetyl erythromycin A with methyl trifluoromethanesulfonate

3.1 g (4 mmol) 2'-acetylerythromycin A was dissolved in 25 ml dry THF with 7 ml DMSO at -13°C. The solution was cooled and stirred while 0.20 g (5 mmol) 60% sodium hydride dispersion in mineral oil was added. Evolution of hydrogen gas was observed and followed using a bubbler until after about 1 hour, gas evolution ceased. Thin layer chromatographic analysis of a sample indicated no decomposition of the intermediate. Upon addition of 0.565 ml (5 mmol) methyl triflouromethane sulfonate, the reaction mixture became exothermic and warmed spontaneously to 0°C. Two ml of TEA were added and the mixture was permitted to warm to 5°C. The reaction mixture was extraced with 200 ml of a 1:1:2 mixture of water, brine and toluene. The layers were separated and the aqueous phase was re-extracted with a mixture of 50 ml hexane and 70 ml toluene. The combined organic portions were washed with two 50 ml aliquots of water and four 50 ml aliquots of brine, and dried over sodium sulfate and magnesium sulfate. The solution was filtered and dried *in vacuo* to yield 2.44 grams of a friable foam which was identified by thin layer chromatography as a mixture of 6-O- and 11-O- methylated materials.

Example VI

The 2'-acetyl-6-O-methylerythromycin A of Example I can be dissolved in methanol, and will spontaneously hydrolyze to 6-O-methylerythromycin A over two days' storage at room temperature.

CLAIMS:

1. A process for O-alkylation of a macrolide antibiotic having a plurality of reactive hydroxyl groups, at positions other than the most reactive hydroxyl group, comprising esterifying the most reactive hydroxyl group, and alkylating one or more of the remaining hydroxyl groups.

2. A process according to Claim 1 wherein the macrolide antibiotic is an erythromycin or oleandomycin compound.

3. A process according to Claim 1 wherein the most reactive hydroxyl group is the 2'-hydroxyl group.

4. A process according to Claim 3 wherein the remaining hydroxyl groups are at the 6, 11, and 4" positions.

5. A process for producing 6-O-,11-O- and 6,11-di-O-alkylated erythromycin compounds, comprising esterifying the 2'-hydroxyl group of erythromycin with an acyl group of the formula

$$X-R-\overset{\overset{O}{\|}}{C}-$$

where R is divalent alkyl, hydroxyalkyl or cycloalkyl or heterocyclic alkyl of 1 to 22 carbon atoms, alkenyl, aryl, alkaryl, cycloaryl or heteroaryl of 3 to 12 carbon atoms and X is hydrogen or a terminal acyl group of the formula:

$$R'-O-\overset{\overset{O}{\|}}{C}-$$

where R' is hydrogen or monovalent alkyl of 1 to 3 carbon atoms, and alkylating the 6-O- position, the 11-O- position, or both.

6. A process according to Claim 5 wherein the esterification is performed by reacting erythromycin with an acylating agent which is selected from compounds of the formulas

(A) $X-R-\overset{O}{\overset{\|}{C}}-Y$, where Y is halogen, R is divalent alkyl, cycloalkyl or heterocyclic alkyl of 1 to 22 carbon atoms, alkenyl, aryl, heteroaryl or alkaryl of 3 to 18 carbon atoms and X is hydrogen or a terminal acyl group of the formula

$R'-O-\overset{O}{\overset{\|}{C}}-$, where R' is monovalent alkyl of 1 to 3 carbon atoms; and

(B) $(R^2-C)_2O$, where $R^2$ is as defined for R, but monovalent.

7. A process according to Claim 6 wherein the esterified erythromycin compound is alkylated by reacting it with an alkyl halide of 1 to 6 carbon atoms in the presence of a strong base.

8. A process according to Claim 7 wherein the base is selected from sodium hydride, potassium hydride, sodium dimsyl, sodium silazide, and lithium silazide.

9. A process according to Claim 8 wherein the alkyl halide is methyl iodide.

European Patent Office

**EUROPEAN SEARCH REPORT**

0177696

Application number

EP 85 10 9538

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.4) |
| Y | EP-A-0 041 355 (TAISHO) * Pages 3,4 * & US - A - 4 331 803 (Cat. D) --- | 1-9 | C 07 H 17/08 |
| Y | EP-A-0 080 818 (TAISHO) * Pages 4,5 * --- | 1-9 | |
| Y | EP-A-0 080 819 (TAISHO) * Pages 3,4 * --- | 1-9 | |
| Y | EP-A-0 114 486 (PFIZER INC.) * Page 5, lines 14-25; page 8 * ----- | 1-9 | |
| | | | TECHNICAL FIELDS SEARCHED (Int. Cl.4) |
| | | | C 07 H 17/00 |

The present search report has been drawn up for all claims

| Place of search | Date of completion of the search | Examiner |
|---|---|---|
| THE HAGUE | 04-12-1985 | VERHULST W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document